# EUROPEAN PATENT SPECIFICATION

(11) **EP 2 640 302 B1**
(45) Date of publication and mention of the grant of the patent: **20.12.2017**
(21) Application number: 11794282.1
(22) Date of filing: 16.11.2011
(51) Int. Cl.: A61B 34/00

(54) **BEARING ASSEMBLY FOR INSTRUMENT**
LAGERANORDNUNG FÜR EIN INSTRUMENT
ENSEMBLE DE SUPPORT POUR INSTRUMENT

(30) Priority: 17.11.2010 US 414854 P
(43) Date of publication of application: 25.09.2013
(73) Proprietor: Boston Scientific Scimed, Inc., Maple Grove, MN 55311-1566 (US)
(72) Inventor: SMITH, Paul, Smithfield, RI 02917 (US); KAPPEL, Gary, Acton, MA 01720 (US); WEITZNER, Barry, Acton, MA 01720 (US); GOLDEN, John, Norton, MA 02766 (US); DALY, Erin, Nashua, NH 03063 (US)
(74) Representative: Vossius & Partner Patentanwälte Rechtsanwälte mbB
(86) International application number: PCT/US2011/060979
(87) International publication number: WO 2012/074751

(56) References cited:
- WO-A1-03/026505
- US-A1- 2008 188 868

## Description

### Field of the Invention

Embodiments of the invention include medical instruments and more particularly bearing assemblies for medical instruments and related methods of use.

### Background of the Invention

Minimally invasive surgical instruments, such as endoscopic and laparoscopic devices, can provide access to surgical sites while minimizing patient trauma. Although the growing capabilities of such therapeutic and diagnostic devices allow physicians to perform an increasing variety of surgeries through traditional minimally invasive routes, further refinements may allow surgical access through even less invasive routes. Currently some robotic systems have been proposed to allow surgical access via a natural orifice. The user interface is remote from surgical instruments and/or end effectors. Unfortunately, these systems are generally expensive and complicated. In addition, they fail to provide the tactile user feedback that traditional devices can provide. Accordingly, there is room for further refinement to conventional minimally invasive surgical devices and a need to develop new surgical systems. An example of tracking tool is shown in Figs. 7 and 8 of patent document WO03/026505A1 Document US-A-2008/0188868 shows a system for guiding an instrument and discloses a frame, and an outer tubular member.

It is to be understood that both the foregoing general description and the following detailed description are exemplary and explanatory only and are not restrictive of the invention.

### SUMMARY OF THE INVENTION

A system for guiding an instrument according to the invention is defined in appended claim 1. According to an embodiment, a system for guiding an instrument may include a frame and a bearing assembly supported by the frame. The bearing assembly includes an outer tubular member supported by the frame and including a proximal end and a distal end. The bearing assembly may also include a bearing at least partially disposed within at least one of the proximal end and the distal end of the outer tubular member. The channel may extend through the outer tubular member and the bearing.

According to another embodiment, a system for guiding an instrument may include a frame and an elongate member supported by the frame. The elongate member may include a proximal end and a distal end. The system may also include a bearing assembly supported by the frame and including a channel for receiving the instrument. The channel may extend between a proximal end of the bearing assembly and a distal end of the bearing assembly. At least a portion of the proximal end of the elongate member may be inserted into the distal end of the bearing assembly.

According to yet another embodiment, a system for guiding an instrument may include a frame including a bearing support. The system may also include a bearing assembly supported by the bearing support and including a channel for receiving the instrument. The channel may extend between a proximal end of the bearing assembly and a distal end of the bearing assembly. The system may further include a drive mechanism connected to the bearing support and configured to drive at least one of the instrument or the bearing assembly axially.

According to a further embodiment, a method for guiding an instrument may include supporting a bearing assembly and an elongate member on a frame. The bearing assembly may be positioned at an angle relative to a proximal end of the elongate member. The method may also include connecting a distal end of the bearing assembly to the proximal end of the elongate member, and inserting the instrument through the bearing assembly and through the elongate member.

Additional objects and advantages of the invention will be set forth in part in the description which follows, and in part will be obvious from the description, or may be learned by practice of the invention. The objects and advantages of the invention will be realized and attained by means of the elements and combinations particularly pointed out below.

It is to be understood that both the foregoing general description and the following detailed description are exemplary and explanatory only and are not restrictive of the invention.

### BRIEF DESCRIPTION OF THE DRAWINGS

The accompanying drawings, which are incorporated in and constitute a part of this specification, illustrate several embodiments of the invention and together with the description, serve to explain the principles of the invention.
Fig. 1 is a perspective view of a proximal end of an endoscopy system including an elongate member, a pair of bearing assemblies, and a pair of instruments, according to an exemplary embodiment;
Fig. 2 is a schematic view of a distal end of an endoscopy system, according to an exemplary embodiment;
Fig. 3 is a top view of the elongate member, bearing assemblies, and instruments of Fig. 1;
Fig. 4 is a top view of an elongate member, a bearing assembly, and an instrument of an endoscopy system, according to another exemplary embodiment;
Fig. 5 is an exploded view of one of the bearing assemblies of Fig. 1;
Fig. 6 is a perspective view of an outer tubular member of the bearing assembly of Fig. 5;
Fig. 7 is a distal end view of the outer tubular member of Fig. 6;
Fig. 8 is a cross-sectional side view of the outer tubular member of Fig. 6;
Fig. 9 is a perspective view of a bearing of the bearing assembly of Fig. 5;
Fig. 10 is a distal end view of the bearing of Fig. 9;
Fig. 11 is a cross-sectional side view of the bearing of Fig. 9;
Fig. 12 is a perspective view of a guide member of the bearing assembly of Fig. 5;
Fig. 13 is a top view of the guide member of Fig. 12;
Fig. 14 is a cross-sectional side view of the guide member of Fig. 12;
Fig. 15 is a cross-sectional distal end view of the guide member of Fig. 12;
Fig. 16 is a distal end view of the guide member of Fig. 12;
Fig. 17 is an exploded view of a frame and a pair of bearing assemblies of an endoscopy system, according to another exemplary embodiment;
Fig. 18 is a perspective view of the frame and the bearing assemblies of Fig. 17;
Fig. 19 is a perspective view of an elongate member inserted into a bearing assembly, according to an exemplary embodiment;
Fig. 20 is a perspective view of an elongate member inserted into a bearing assembly, according to another exemplary embodiment;
Fig. 21 is a perspective view of a bearing support and a bearing assembly, according to an exemplary embodiment;
Fig. 22 is a perspective view of a bearing support and a bearing assembly, according to another exemplary embodiment;
Fig. 23 is a perspective view of a bearing support and a bearing assembly, according to a further exemplary embodiment;
Fig. 24 is a perspective view of a frame and various interchangeable bearing assemblies of the endoscopy system of Fig. 18;
Fig. 25 is a cross-sectional side view of a locking mechanism for locking an instrument with respect to a bearing assembly, according to an exemplary embodiment;
Fig. 26 is a cross-sectional side view of a locking mechanism for locking an instrument with respect to a bearing assembly, according to another exemplary embodiment;
Fig. 27 is a perspective view of a proximal end of an endoscopy system including a pair of bearing assemblies and a pair of instruments, according to another exemplary embodiment;
Fig. 28 is a perspective view of a bearing assembly, according to a further exemplary embodiment; and
Fig. 29 is a perspective view of a proximal end of an endoscopy system including a pair of bearing assemblies and a pair of instruments, according to another exemplary embodiment.

### DESCRIPTION OF THE EMBODIMENTS

Reference will now be made in detail to exemplary embodiments of the invention, examples of which are illustrated in the accompanying drawings. Wherever possible, the same reference numbers will be used throughout the drawings to refer to the same or like parts.

The terms "proximal" and "distal" are used herein to refer to the relative positions of the components of the exemplary endoscopy system 10. When used herein, "proximal" refers to a position relatively closer to the exterior of the body or closer to the surgeon using the endoscopy system 10. In contrast, "distal" refers to a position relatively further away from the surgeon using the endoscopy system 10 or closer to the interior of the body.

In addition, while the discussion of systems and methods below may generally refer to "surgical instruments," "surgery," or a "surgical site" for convenience, the described systems and their methods of use are not limited to tissue resection and/or repair. In particular, the described systems may be used for inspection and diagnosis in addition, or as an alternative, to surgical treatment. The treatment is not limited to any particular treatment. Various other exemplary treatment devices and methods are referred to herein. Moreover, the systems described herein may perform non-medical applications such as in the inspection and/or repair of machinery.

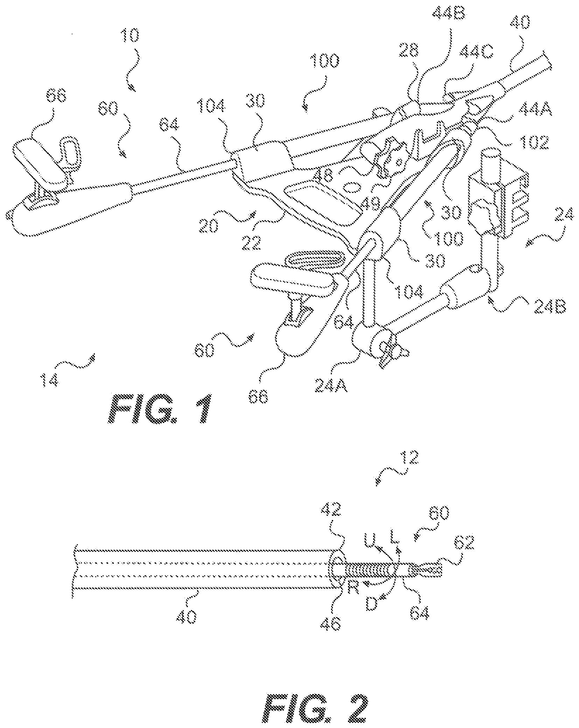

Figs. 1 and 2 depict an exemplary endoscopy system 10 that may be used for any therapeutic or diagnostic endoscopic procedure and the components thereof. The phrase "endoscopic procedure" is broadly used to indicate any medical procedure that may be performed by inserting an endoscope, guide tube, catheter, or any other medical device into the body through any anatomic opening. The endoscopy system 10 may be used for performing surgery at a relative distance via medical instruments including or directly connected to user controls. The endoscopy system 10 may be adapted for trans-oral, trans-anal, trans-vaginal, trans-urethral, trans-nasal, transluminal, laparoscopic, thorascopic, orthopedic, through the ear, and/or percutaneous access. For example, the components of the endoscopy system 10 described below may be made of any suitable material capable of being inserted into the body, e.g., a suitable biocompatible material.

The endoscopy system 10 includes a proximal end 14 (Fig. 1) and a distal end 12 (Fig. 2). At the proximal end 14, the endoscopy system 10 may include a frame 20 for supporting and positioning various components of the endoscopy system 10, such as an elongate member 40 (e.g., a guide tube), one or more instruments 60, and one or more bearing assemblies 100 that guide and/or support the instruments 60 as described below.

The frame 20 may have a variety of configurations depending on patient location, spacing, ergonomics, user preference, and/or the availability of operating table space. As shown in Fig. 1, the frame 20 may include a platform 22 that may include a generally planar (e.g., horizontal) surface supported by an adjustable leg 24 or other type of support that may rest on or may be attached to, e.g., the floor, an operating table surface or rail, or other surface or rail. The adjustable leg 24 may include a hinge 24a, a telescoping section (not shown), a sliding member 24b that may be locked in place at desired positions, or other device configured to modify the position, configuration, and/or orientation (translational, rotational, etc.) of the platform 22 and/or other components of the frame 20 with respect to the ground, an operating table, or other base surface.

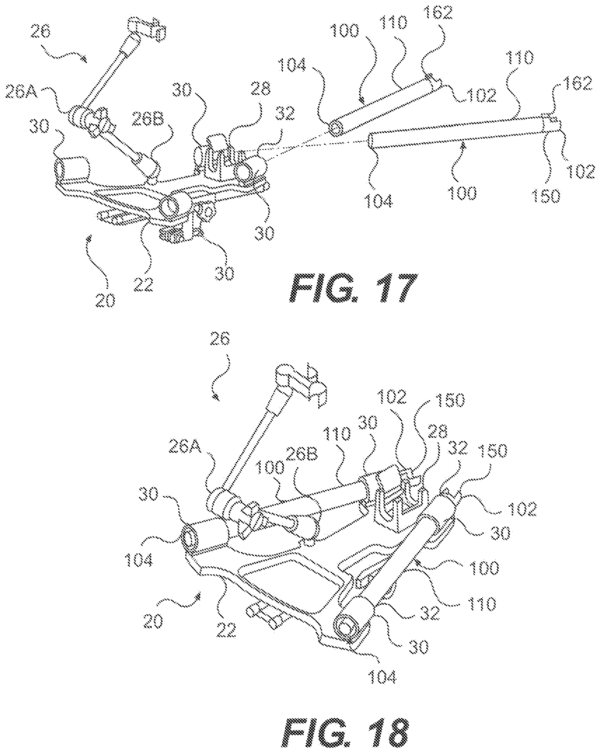

The frame 20 may further include an adjustable arm 26 (Figs. 17 and 18) for supporting an endoscope (not shown) or other component of the endoscopy system 10. The adjustable arm 26 may be supported by the platform 22 and may include a hinge 26a, a pivotal connection 26b, a telescoping section (not shown), a sliding member (not shown) that may be locked in place at desired positions, or other device configured to modify the position, configuration, and/or orientation (translational, rotational, etc.) of the endoscope or other component with respect to the platform 22.

As shown in Fig. 1, the frame 20 may also include an elongate member support 28 for supporting the elongate member 40. The elongate member support 28 may be supported by the platform 22 and may include a slot or surface, e.g., with a generally U-shaped cross-section, to receive and support the elongate member 40. The frame 20 may also include an adjustment mechanism configured to adjust the position (translational, rotational, etc.) of the elongate member support 28 with respect to the platform 22 and/or may include a locking mechanism to lock the elongate member support 28 in place with respect to the platform 22 or to lock the elongate member 40 within the elongate member support 28.

The elongate member 40 includes at least one distal end 42 (Fig. 2), and at least one proximal end 44a-44c (Figs. 1, 18, and 19). As shown in Fig. 1, the exemplary elongate member 40 branches into proximal ends 44a-44c. As shown, proximal ends 44a-b can diverge to mate with the distal ends of bearing assemblies 100. In other embodiments, bearing assembly 100 could include an angled distal end configured to engage a non-diverging proximal end of elongate member 40. Bearing assembly 100 and elongate member 40 can be variously mated to provide for movement of instrument 60 between the two structures.

In some embodiments, two proximal ends 44a-c can connect to respective bearing assemblies 100. One proximal end 44a-c can extend proximally between the bearing assemblies 100, and may include a port for receiving an endoscope, colonscope, or other instrument (not shown), which may be supported by the adjustable arm 26. One proximal end 44a-c can include a port, e.g., for injecting fluids, insufflation, inserting an additional instrument, etc. As shown in Fig. 2, the exemplary elongate member 40 includes one distal end 42. Alternatively, the elongate member 40 may include more than one distal end 42, e.g., to separately position two instruments 60 at different locations within a patient.

The elongate member 40 may include at least one channel 46 (Fig. 2) extending substantially longitudinally (axially) within the elongate member 40, generally between the distal end(s) 42 and the proximal end(s) 44a-44c of the elongate member 40. For example, elongate member 40 could include a first channel configured to receive a first instrument and a second channel configured to receive a second instrument. As shown in Figs. 1 and 2, proximal ends 44a-44c of the exemplary elongate member 40 may include channels that connect to a single channel 46 that extends along the length of the elongate member 40 and forms an opening in the single distal end 42. Thus, separate instruments may be inserted through the respective proximal ends 44a-44c, advanced through the same channel 46 in the elongate member 40, and through the opening shown in Fig. 2 formed by the channel 46 in the distal end 42 of the elongate member 40.

Alternatively, one or more of the proximal ends 44a-44c shown in Fig. 1 may include channels that connect to respective channels 46 that extend along the length of the elongate member 40 and form respective openings in the single distal end 42 (or in respective distal ends 42). Thus, separate instruments may be inserted through the separate proximal ends 44a-44c, advanced through separate channels 46 in the elongate member 40, and through different openings formed by the channels 46 in the distal end(s) 42 of the elongate member 40.

The distal end 42 of the elongate member 40 may be configured to be advanced through any body cavity or body lumen of a patient. For example, the distal end 42 may have a tapered and/or atraumatic distal end configuration. The elongate member 40 may be flexible, for example, to be able to traverse tortuous anatomy. Embodiments may be applicable to applications where a medical device is inserted into the body through an anatomic opening (e.g., an incision or a natural orifice). For example, embodiments of the current disclosure may be used in, but are not limited to, natural orifice transluminal endoscopic surgery (NOTES) procedures or single incision laparoscopic surgical (SILS) procedures. The elongate member 40 may be configured to be advanced through any body lumen, e.g., the gastrointestinal tract (GI tract), the pulmonary tract, the anal tract, etc. For example, the elongate member 40 may access the abdominal cavity trans-orally, trans-vaginally, trans-anally, or through a single incision approach through the GI tract or umbilicus in order to access organs within the abdominal cavity.

Thus, the distal end 42 of the elongate member 40 may be positioned internal to the body, and the proximal ends 44a-44c of the elongate member 40 may be positioned external to the body near the frame 20 in the proximal end 14 of the endoscopy system 10, as shown in Fig. 1. The elongate member 40 may include imaging and light capabilities, and may also include structure for steering the distal end 42 of the elongate member 40 and/or locking the distal end 42 of the elongate member 40 in position, e.g., after steering the distal end 42. That structure may include rotatable control knobs 48, 49 positioned at a proximal handle of the elongate member 40. The knobs 48, 49 may connect control wires or cables (not shown) within the elongate member 40, to provide up/down and left/right steering of the distal end 42 of the elongate member 40. Alternatively, the elongate member 40 may be formed of a material that is bendable and conforms to the endoscope or other instrument inserted into the channel 46 as the endoscope or other instrument is steered.

The frame 20 may also include at least one bearing support 30 for supporting at least one bearing assembly 100. The bearing support 30 may be supported by the platform 22 and may be sized to receive and support the bearing assembly 100. For example, as shown in Fig. 1, the exemplary bearing support 30 includes a generally cylindrical inner surface for receiving and supporting the generally cylindrical bearing assembly 100. Other inner surface and assembly shapes are also contemplated. Also, as shown in Fig. 1, the exemplary endoscopy system 10 includes a pair of bearing assemblies 100, each including a distal end 102 and a proximal end 104, and a pair of bearing supports 30 supporting each bearing assembly 100 at the respective distal and proximal ends 102, 104. Alternatively, each bearing assembly 100 may be supported by a single bearing support 30 or more than two bearing supports 30, and/or the endoscopy system 10 may include one bearing assembly 100 or more than two bearing assemblies 100. The frame 20 may also include an adjustment mechanism configured to adjust the position (translational, rotational, etc.) of each bearing support 30 with respect to the platform 22 and/or may include a locking mechanism to lock each bearing support 30 in place with respect to the platform 22 or to lock the bearing assembly 100 within the respective bearing support 30. In some embodiments, bearing assembly 100 may be disposable and frame 20 may be reusable.

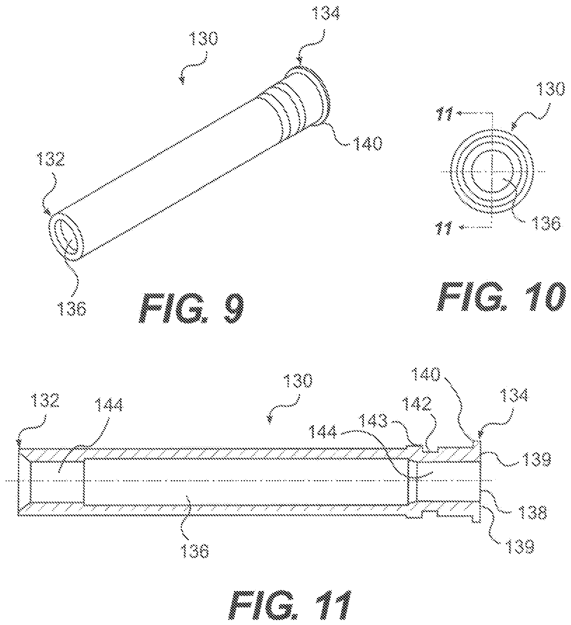

The distal end 102 of the bearing assembly 100 connects to the proximal end 44a of the elongate member 40 as will be described below. The proximal end 104 includes an opening 138 (Fig. 11) for receiving the distal end of the instrument 60. To position the instrument 60 at a working location inside the patient for an endoscopic procedure, the distal end 42 of the elongate member 40 may be inserted into an opening in the patient, advanced into the patient (e.g., advanced into a body organ, through a body lumen, etc.), and the elongate member 40 may be attached to the frame 20 using the elongate member support 28. The instrument 60 may be advanced through the proximal end 104 of the bearing assembly 100, axially through the bearing assembly 100, through the connection between the bearing assembly 100 and the elongate member 40, through the elongate member 40, and through the distal end 42 of the elongate member 40.

Fig. 2 shows the distal end of one of the two instruments 60 shown in Fig. 1 that is advanced through the elongate member 40. Although not shown in Fig. 2, the distal ends of both instruments 60 of Fig. 1 may be advanced through the distal end 42 of the elongate member 40. The instrument 60 may include an end effector 62 attached to a distal end of an elongate member 64. The end effector 62 may include a device configured to assist in performing an endoscopic or surgical procedure. For example, the end effector 62 may include, but is not limited to, a cutting device (e.g., scissors, tissue cutter, etc.), forceps, a fixation device, a manipulation device, a dissection device, a support device, a sealing device, a needle holder, a cautery device, a closure device (e.g., clips, staples, loops, ligator, suturing device, etc.), a retrieval device (e.g., snare, basket, loop, a fluid extraction device, etc.), a tissue exploration device (e.g., optical device, illumination device, etc.), a tissue sampling or biopsy device, a needle, an injection device, an aspiration device, any device with pivoting jaws, a delivery device, a device for aiding in the patency of a lumen or for dilating an opening (e.g., a balloon or other expandable member, stent, wire structure, etc.), and/or a grasping device, etc. Accordingly, the instrument 60 and the end effector 62 may be any type of suitable instrument and end effector known to those skilled in the art. Some exemplary configurations of elongate members and instruments are disclosed, for example, in U.S. Patent Application Publication No. 2008/0188890, entitled "Multi-Part Instrument Systems and Methods" and U.S. Patent Application Publication No. 2008/0188868, entitled "Direct Drive Endoscopy Systems and Methods,".

The instrument 60 may include a handle portion or control device 66 near the proximal end of the instrument 60. The control device 66 may be connected to the end effector 62 via cables, bare wires, insulated wires or cables, other elongate flexible members, or other devices for connecting the control device 66 to the end effector 62. The control device 66 may also have suitable connections for cautery, insufflation, irrigation, or vacuum devices of the instrument 60 and/or the end effector 62. The control device 66 allows the user to control the end effector 62, such as the articulation (e.g., orientation, position, movement, etc.) and functionality of the end effector 62. For example, the control device 66 may control the end effector 62 to move the end effector 62 longitudinally, laterally, and/or rotationally. As indicated in Fig. 2, moving the end effector 62 laterally may include articulating or moving the end effector 62 up (U), down (D), left (L), and/or right (R), for example, with respect to an adjacent portion of the instrument 60. The control device 66 may also control the functionality of the end effector 62, such as by initiating a function or action of the end effector 62, e.g., opening, closing, etc. Thus, the control device 66 allows the user to control one or more degrees of freedom, e.g., up/down and/or left/right movement, of the end effector 62, and allows the user to control the movement and operation of the instrument 60 with a single hand, which may free the user's other hand to control other devices or instruments. Some exemplary control devices are disclosed, for example, in U.S. Patent Application Publication No. 2007/0010801 and U.S. Patent Application Publication No. 2008/0287862.

The instrument 60 may be bent or articulated into a desired configuration to perform a procedure. The instrument 60 may be flexible, rigid, bendable, straight, malleable, etc., and may include sections of different degrees of flexibility/rigidity. For example, a distal portion of the elongate member 64 of the instrument 60 may be relatively flexible and/or more flexible than a proximal end portion to allow the instrument 60 to be slidably inserted into and through the elongate member 40. Stiffness may be altered or controllable at any location along instrument 60. The flexible distal portion allows the instrument 60 to pass through passageways that are not straight, such as the connection between the proximal ends 44a-44c of the elongate member 40 and the channel 46, where the proximal ends 44a-44c are located at angles with respect to the channel 46.

The proximal portion of the elongate member 64 of the instrument 60 may be relatively rigid and/or more rigid than the distal portion, and the rigid proximal portion may be received in the bearing assembly 100, e.g., may include substantially the entire length of the instrument 60 inserted in the bearing assembly 100. Guiding the rigid proximal portion with the bearing assembly 100 may allow easier control of the movement of the end effector 62 of the instrument 60 since the rigid proximal portion may be less likely to be floppy or to bend when contacting the bearing assembly 100 and may be less likely to kink or twist within the bearing assembly 100. Further, supporting the rigid proximal portion in the bearing assembly 100 may allow the user to leave the instrument 60 in place within the bearing assembly 100 when the user releases the instrument 60.

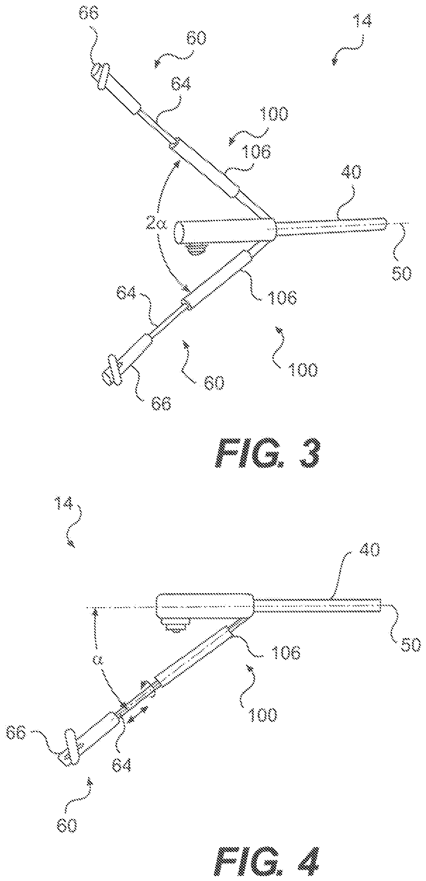

As shown in Figs. 3 and 4, each bearing assembly 100 may include a longitudinal axis 106, and the elongate member 40 may include a longitudinal axis 50. Each bearing assembly 100 may be positioned so that the respective longitudinal axis 106 diverges away from the longitudinal axis 50 of the elongate member 40. For example, an angle α separating the longitudinal axes 50, 106 may range from, but not be limited to, 10 degrees to 60 degrees. The angle α may depend on various factors, such as user comfort and other ergonomic considerations, possible interference between the control devices 66 of the instruments 60 or other components of the endoscopy system 10, etc. The positioning (translational, rotational, etc.) of the respective bearing assemblies 100 may be adjustable with respect to each other and/or the elongate member 40 such that the respective angles α separating each longitudinal axis 106 of the bearing assembly 100 from the longitudinal axis 50 of the elongate member 40 are adjustable or selectively lockable. The positioning (translational, rotational, etc.) of the longitudinal axis 50 of the elongate member 40 may also be adjustable to change the respective angles α.

Figs. 3 and 4 depict the angles α in a single plane, e.g., a generally horizontal plane. Alternatively, the bearing assemblies 100 may be positioned so that the angles α are in a different plane or different respective planes. As another embodiment, an additional bearing assembly 100 (e.g., a third, fourth, etc.) may be provided and may have a longitudinal axis positioned at an angle with respect to the longitudinal axis 50 of the elongate member 40 that is in the generally horizontal plane and/or a different plane than the generally horizontal plane.

In the exemplary embodiment of the endoscopy system 10 having two bearing assemblies 100 shown in Fig. 3, the bearing supports 30 supporting the bearing assemblies 100 may be positioned with respect to the elongate member 40 so that the bearing assemblies 100 diverge away from each other toward the proximal end 14 of the endoscopy system 10. The longitudinal axis 106 of each bearing assembly 100 may be separated by the angle α from the longitudinal axis 50 of the elongate member 40, and an angle of 2α may separate the longitudinal axes 106 from each other. Bearing assembly 100 may also be selectively lockable.

In the exemplary embodiment of an endoscopy system having a single bearing assembly 100 shown in Fig. 4, the bearing supports 30 are positioned with respect to the elongate member 40 so that the longitudinal axis 106 of the bearing assembly 100 may be separated by the angle α from the longitudinal axis 50 of the elongate member 40.

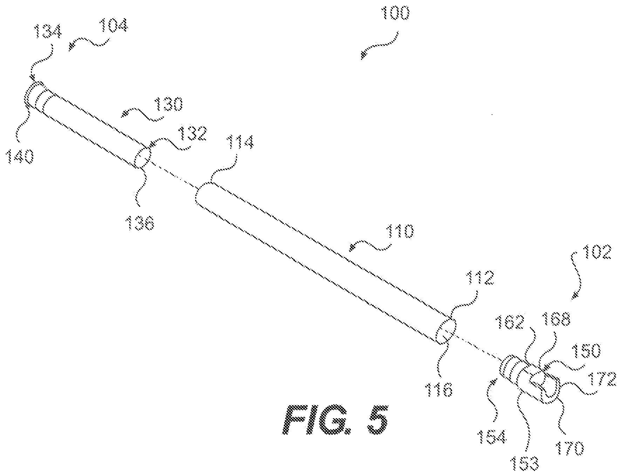

Figs. 5-20 show the assembly of the bearing assemblies 100 onto the frame 20, according to an exemplary embodiment. As shown in Fig. 5, the exemplary bearing assembly 100 includes an outer tubular member 110, a bearing 130, and a guide member 150. The bearing 130 and the guide member 150 are inserted into and positioned at opposite ends 112, 114 of the outer tubular member 110, respectively. The bearing 130 is located at the proximal end 104 of the bearing assembly 100, and the guide member 150 is located at the distal end 102 of the bearing assembly 100.

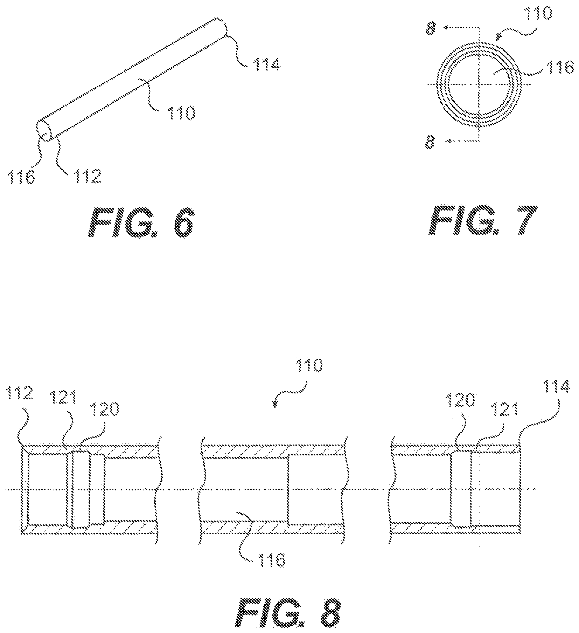

Figs. 6-8 show the outer tubular member 110, which includes a distal end 112, a proximal end 114, and a channel 116 extending substantially longitudinally within with outer tubular member 110 between the distal end 112 and the proximal end 114. In an embodiment, the outer tubular member 110 may be approximately 11-13 inches long. As shown in Fig. 8, the inner surface of the outer tubular member 110 defining the channel 116 may include one or more inner grooves 120 and/or one or more inner protrusions 121 for engaging with corresponding features of the bearing 130 and the guide member 150 to position the bearing 130 and the guide member 150 axially within the outer tubular member 110. The inner surface can also include ribbing, which may include spiral ribbing. The outer tubular member 110 is sized to be inserted into the bracket support 30. For example, as shown in Figs. 6-8, the outer tubular member 110 may have a circular outer cross-section sized to be received in an opening in the bracket support 30 having a circular cross-section. The bracket support 30 may include a locking mechanism (not shown) to fix the outer tubular member 110 in place, e.g., rotationally and axially, inside the bracket support 30.

The outer tubular member 110 may be formed of a material or have a coating that provides a relatively frictionless surface in the channel 116 against which the instrument 60 may slide. The portion of the inner surface of the outer tubular member 110 against which the instrument 60 may slide may have a cross-section that is sized to correspond to the portion of the instrument 60 that slides through the outer tubular member 110.

Also, as shown in Fig. 1, the outer tubular member 110 may be made of a clear, transparent, semitransparent, and/or translucent material, e.g., polycarbonate, Lexan, or other polymer, plastic, etc. Alternatively, a portion of the outer tubular member 110 may be formed of such a material, e.g., to form a window. As a result, the outer tubular member 110 may allow the user to view the interior of the bearing assembly 100 while maintaining a protective barrier around the instrument 60 that prevents fluids (e.g., biological fluids or insufflation fluids) from escaping. For example, the user may view the outer surface of the instrument 60 inserted through the bearing assembly 100, and the outer surface of the instrument 60 may have markers that indicate, for example, the distance to the distal end of the instrument 60, thereby also indicating how far into the patient the instrument 60 has advanced.

Figs. 9-11 show the bearing 130, which includes a distal end 132, a proximal end 134, and a channel 136 extending substantially longitudinally within with bearing 130 between the distal end 132 and the proximal end 134. In an embodiment, the bearing 130 may be approximately 4-6 inches long. The distal end of the instrument 60 may be inserted into the bearing assembly 100 through the opening 138 of the channel 136 at the proximal end 134. The opening 138 may be formed with a chamfer 139 that may smooth and/or facilitate the transition of the instrument 60 into the bearing assembly 100 and that may also assist in preventing the snagging of a user's gloves or other materials when inserting the instrument 60 into the bearing assembly 100.

The bearing 130 may include a proximal flange 140 configured to abut against or otherwise engage the proximal end 114 of the outer tubular member 110. As a result, the proximal flange 140 assists in positioning the bearing 130 at the proximal end 114 of the outer tubular member 110.

The outer surface of the bearing 130 includes one or more outer grooves 142 and/or one or more outer protrusions 143 for engaging with respective inner protrusions 121 and/or inner grooves 120 of the outer tubular member 110. These features may engage to fix the bearing 130 in place axially inside the outer tubular member 110 at the proximal end 104 of the bearing assembly 100 and to assist in preventing the bearing 130 from sliding axially with respect to the outer tubular member 110.

The bearing 130 may be formed of a material or have a coating that provides a relatively frictionless surface in the channel 136 against which the instrument 60 may slide. For example, the bearing 130 may be formed of a low friction or "non-stick" material, e.g., Teflon, polytetrafluoroethylene (PTFE), or other polymer, plastic, etc. The material may have a relatively low coefficient of friction compared to, for example, the material used to form the outer tubular member 110 and/or the guide member 150. As a result, the bearing 130 may reduce surface friction between the instrument 60 and the bearing assembly 100 as the instrument 60 slides longitudinally or rotationally within the bearing assembly 100. In an embodiment, in addition to the outer tubular member 110, the bearing 130 may include a clear, transparent, semitransparent, and/or translucent material, e.g., polycarbonate or other polymer, plastic, etc., to allow a user to see inside the bearing 130.

The length and inner diameter of the bearing 130 may be determined to provide a smooth transition of the instrument 60 into the bearing assembly 100 and to minimize the resistance to movement (surface friction) of the instrument 60 within the bearing assembly 100. The inner surface of the bearing 130 serves as a supporting surface to guide and support flexible and rigid instruments 60, and is sufficiently rigid to withstand user loads that are applied to the instrument 60, e.g., via the control device 66.

The inner diameter of the bearing 130 at the distal and proximal ends 132, 134 may be smaller compared to the inner diameter of the bearing 130 at a middle portion between the distal and proximal ends 132, 134. The smaller inner diameters at the distal and proximal ends 132, 134 form constrictions 144 at the distal and proximal ends 132, 134. The amount of reduction of the inner diameter at the constrictions 144 and the distance between the constrictions 144 may be determined to allow the bearing 130 to withstand user loads (e.g., rotation, longitudinal movement, lateral movement, etc.) that may be applied to the instrument 60. Alternatively, or in addition, the inner diameter of the constrictions 144 in the bearing 130 may have a cross-section that is sized to correspond to the portion of the instrument 60 that slides through the bearing 130.

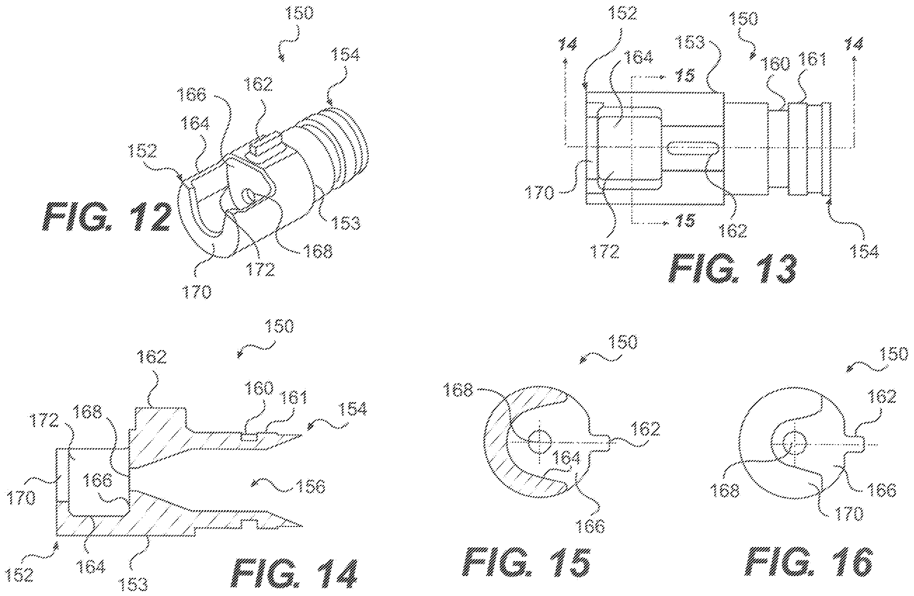

Fig. 12-16 show the guide member 150, which includes a distal end 152, a proximal end 154, and a channel 156 extending substantially longitudinally within the guide member 150 between the distal end 152 and the proximal end 154. In an embodiment, the guide member 150 may be approximately 1-3 inches long. The guide member 150 may be formed of a material or have a coating that provides a relatively frictionless surface in the channel 156 against which the instrument 60 may slide. The guide member 150 may be formed from Teflon (PTFE), acetal, Celcon, polyoxymethylene, or other plastic or polymer. In an embodiment, in addition to the outer tubular member 110, the guide member 150 may be made of a clear, transparent, semitransparent, and/or translucent material, e.g., polycarbonate or other polymer, plastic, etc., to allow a user to see inside the guide member 150.

The outer surface of the proximal end 154 of the guide member 150 includes one or more outer grooves 160 and/or one or more outer protrusions 161 for engaging with respective inner protrusions 121 and/or inner grooves 120 of the outer tubular member 110. These features may engage to fix the guide member 150 in place axially inside the outer tubular member 110 at the distal end 102 of the bearing assembly 100 and to assist in preventing the guide member 150 from sliding axially with respect to the outer tubular member 110.

The guide member 150 may also include an intermediate portion 153 between the distal and proximal ends 152, 154. A key 162 or projection may extend outwardly from an outer surface of the intermediate portion 153. The key 162 may be shaped to be received within a corresponding keyway 32 (Figs. 17 and 18) in the distal end of the bearing support 30 in which the distal end 102 of the bearing assembly 100 is received. Alternatively, the guide member 150 may include the keyway 32 and the bearing support 30 may include the key 162, or other types of engaging features may be used. In an embodiment, the key 162 may sized to form a friction or interference fit with the keyway 32. Positioning the key 162 in the keyway 32 serves to locate the bearing assembly 100 with respect to the bearing support 30 and the frame 20. When the key 162 is inserted into the keyway 32, the key 162 positions the bearing assembly 100 rotationally and axially within the bearing support 30.

In the embodiment shown in Figs. 12-16, the distal end 152 of the guide member 150 includes a U-shaped surface 164 extending distally from a distal surface 166 of the intermediate portion 153. The distal surface 166 of the intermediate portion 153 includes an opening 168 through which the instrument 60 may pass to exit the bearing assembly 100. In the embodiment shown in Figs. 12-16, the U-shaped surface 164, the distal surface 166 of the intermediate portion 153, and an end flange 170 at the distal end 152 of the guide member 150 define a cavity 172 for receiving the proximal end 44a of the elongate member 40 as will be described below. Other shaped surfaces are also contemplated.

Figs. 17 and 18 show the attachment of the bearing assembly 100 to the frame 20. First, the bearing 110 and the guide member 150 may be inserted into the outer tubular member 130 (Fig. 9) to form the bearing assembly 100. Then, as shown in Fig. 17, the bearing assembly 100 may be inserted through the bearing supports 30 until the key 162 in the guide member 150 of the bearing assembly 100 is received in the keyway 32 in the bearing support 30 in which the distal end 102 of the bearing assembly 100 is positioned. Fig. 18 shows the bearing assemblies 100 positioned in the respective bearing supports 30 with the keys 162 engaged with the respective keyways 32. Then, the elongate member 40 may be attached to the bearing assembly 100. In other embodiments, guide member 150 can attach without a key. For example, guide member 150 may be attached via a thread, snap, lock-in, latch, or similar attachment device.

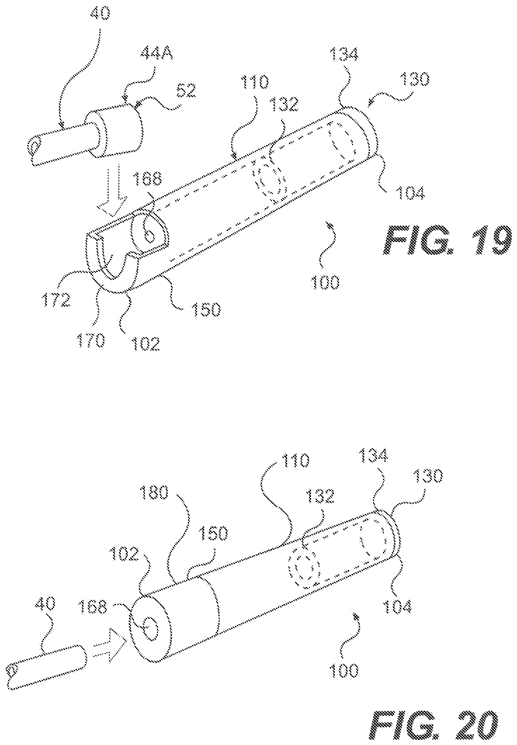

Fig. 19 shows the attachment of the proximal end 44a of the elongate member 40 to the bearing assembly 100. The proximal end 44a of the elongate member 40 may be attached to the bearing assembly 100 while the bearing assembly 100 is positioned in the bearing supports 30 as shown in Fig. 18. Alternatively, the elongate member 40 may be attached to the bearing assembly 100 before the bearing assembly 100 is positioned in the bearing supports 30.

As shown in Fig. 19, the proximal end 44a of the elongate member 40 may include a seal 52. The seal 52 may be formed of an elastomeric material, such as rubber, silicone, or other materials known in the art for forming seals. In some instances, a soft or compliant foam may be lubricated to reduce friction or improve sealing. The seal 52 may have a larger outer dimension (e.g., outer diameter) than the majority of elongate member 40. The seal 52 may be inserted laterally into the cavity 170 in the guide member 150 to attach the proximal end 44a of the elongate member 40 to the distal end 102 of the bearing assembly 100. When the elongate member 40 is attached to the bearing assembly 100, the opening 168 in the guide member 150 aligns with the channel 46 in the elongate member 40 to allow the instrument 60 to pass through the bearing assembly 100 into the elongate member 40, thereby smoothing the transition for the instrument 60 as it passes from the guide member 150 to the elongate member 40. Since the seal 52 is inserted laterally into the cavity 172 and includes a larger outer dimension than the opening in the end flange 170 of the guide member 150, the seal 52 may be prevented from slipping axially through the opening in the end flange 170 and may be held in place between the distal surface 166 of the intermediate portion 153 of the guide member 150 and the end flange 170. As a result, the connection between the elongate member 40 and the bearing assembly 100 is less likely to unintentionally separate. Also, the seal 52 assists in preventing fluids (e.g., biological fluids or insufflation fluids from the instrument 60) from escaping from within the elongate member 40 and the bearing assembly 100. Both ends of bearing assembly 100 could include one or more seals or valves.

After connecting the elongate member 40 to the bearing assembly 100 and after positioning the bearing assembly 100 on the frame 20, the instrument 60 may be inserted into the bearing assembly 100 through the opening 138 of the channel 136 at the proximal end 134 of the bearing 130. Then, the instrument 60 may pass through the bearing assembly 100, e.g., through the bearing 130, then through the length of the outer tubular member 110 extending between the bearing 130 and the guide member 150, through the proximal end 154 and intermediate portion 153 of the guide member 150, through the opening 168 in the distal surface 166 of the intermediate portion 153 of the guide member 150, through the seal 52, and then through the elongate member 40. The channels 116, 136, 156 in the respective outer tubular member 110, the bearing 130, and the guide member 150 communicate with each other to form a single channel within the bearing assembly 100 through which the instrument 60 may pass.

Fig. 20 shows the attachment of the elongate member 40 to the bearing assembly 100, according to an alternate embodiment. In this embodiment, the proximal end 42 of the elongate member 40 may not include the seal 52 with the larger outer dimension shown in Fig. 19, and the guide member 150 may not include the cavity 172. Instead, the distal end 152 of the guide member 150 may include the opening 168 and a seal 180. The seal 180 may be formed of an elastomeric material, such as rubber or other materials known in the art for forming seals. Any seal may also include a valve, membrane, or coating. The proximal end 44a of the elongate member 40 may be inserted through the seal 180 in the guide member 150 to attach the elongate member 40 to the bearing assembly 100. In an embodiment, the seal 180 may include a membrane with an opening (e.g., a slit) through which the proximal end 44a of the elongate member 40 may pass, and the seal 180 may prevent fluids (e.g., biological fluids or insufflation fluids from the instrument 60) from escaping from within the elongate member 40 and the bearing assembly 100. The membrane may also be included in the opening 168 in the embodiment shown in Fig. 19.

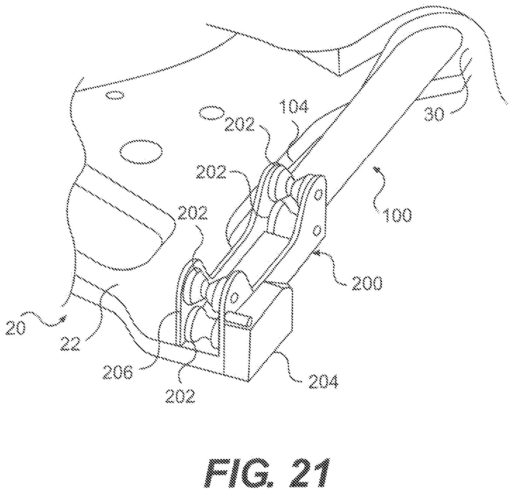

Fig. 21 shows an alternate embodiment of a bearing support 200 of the frame 20 that may be provided instead of, or in addition to, the generally cylindrical bearing support 30 shown in Fig. 1. For example, the frame 20 may include the bearing support 200 shown in Fig. 21 for supporting the proximal end 104 of the bearing assembly 100 while the bearing support 30 shown in Fig. 1 may be provided to support the distal end 102 of the bearing assembly 100, as shown in Fig. 21.

The bearing support 200 may include at least one roller 202 configured to move the bearing assembly 100 and/or the instrument 60 axially. For example, in the embodiment shown in Fig. 21, the bearing support 200 includes two sets of rollers 202, and each set of rollers 202 includes at least one roller 202 that may contact a bottom surface of the bearing assembly 100 or the instrument 60, and at least one roller 202 that may contact a top surface of the bearing assembly 100 or the instrument 60. The size of the rollers 202 may be adjustable to accommodate bearing assemblies 100 or instruments 60 of various sizes. For example, the rollers 202 may be inflatable, and the amount of inflation of the rollers 202 may depend on the size of the bearing assembly 100 or the instrument 60 received in the bearing support 200. In other embodiments, the position of the rollers 202 may be selectively adjustable. In some embodiments, ball bearings could be used in place of rollers 202.

The rollers 202 may be connected to a drive mechanism, such as a motor 204, that rotates the rollers 202 to move the bearing assembly 100 and/or the instrument 60 axially (distally or proximally). The drive mechanism may include a manual drive system that may operate using direct mechanical movement of a knob, lever, or similar other device. The motor 204 may be connected to a sensor 206 that senses an axial position of the bearing assembly 100 and/or the instrument 60. Sensor 206 may also operate without motor 204.

For example, in the embodiment where the rollers 202 contact and move the bearing assembly 100, the sensor 206 may sense whether the bearing assembly 100 is at its farthest possible proximal location and/or its farthest possible distal location to prevent the bearing assembly 100 from inadvertently falling out of the bearing support 200. A controller (not shown) may be provided to control the operation of the motor 204 and to determine when to move the bearing assembly 100 axially based on the sensed axial position of the bearing assembly 100. In the embodiment where the rollers 202 contact and move the instrument 60, the motor 204 may be connected to the sensor 206 to sense a position of the instrument 60, e.g., when the instrument 60 is ready to be loaded and/or removed from the bearing assembly 100. The motor 204 may rotate the rollers 202 to move the instrument 60 axially to insert or remove the instrument 60 from the bearing assembly 100. As a result, the bearing support 200 may facilitate alignment of the instrument 60 with the bearing assembly 100. A controller (not shown) may be provided to control the operation of the motor 204 and to determine when to move the instrument 60 axially based on the sensed axial position of the instrument 60.

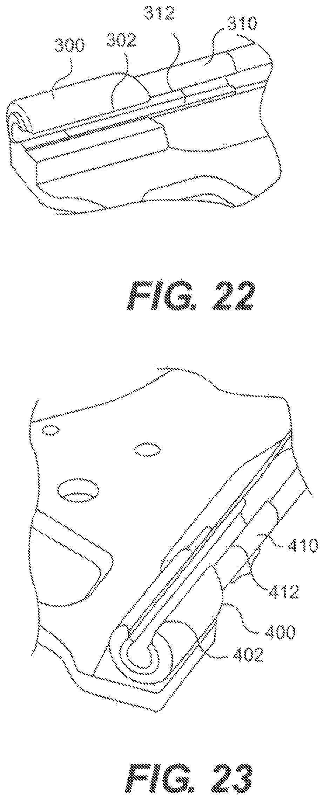

The size and configuration of the bearing support 30 and/or the bearing assembly 100 may vary, e.g., depending on a size and configuration of the instrument 60. Fig. 22 shows an alternate embodiment of a bearing support 300 of the frame 20 and an outer tubular member 310 of the bearing assembly 100, which allow the control device 66 of the instrument 60 to be received within the bearing support 300 and the outer tubular member 310. The bearing support 300 may be provided instead of, or in addition to, the generally cylindrical bearing support 30 shown in Fig. 1. For example, the frame 20 may include the bearing support 300 shown in Fig. 22 for supporting the proximal end 104 of the bearing assembly 100 while the bearing support 30 shown in Fig. 1 may be provided to support the distal end 102 of the bearing assembly 100.

The bearing support 300 may be generally C-shaped or U-shaped such that the bearing support 300 forms a slot 302. The bearing assembly 100 may include a corresponding slot 312 that has similar dimensions (e.g., width and length) as the slot 302 in the bearing support 300. The slot 312 in the bearing assembly 100 may be formed in the outer tubular member 310 and in the bearing 130. The slots 302, 312 may be formed in a side portion of the respective bearing support 300 and bearing assembly 100, as shown in Fig. 22. Accordingly, an instrument 60 having a control device 66 that extends to the side may be inserted into the bearing assembly 100 such that the sideward-extending control device 66 passes through the slots 302, 312. Thus, although Fig. 1 shows that the elongate members 64 of the instruments 60 may be received in the respective bearing assemblies 100, the bearing assemblies 100 and the bearing supports 300 may also be configured to receive the control device 66 of the respective instruments 60. Also, the bearing assemblies 100 and bearing supports 300 allow easy loading of the respective instruments 60 even when the control device 66 of the respective instruments 60 is not passed through the slots 302, 312.

Fig. 23 shows an alternate embodiment of a bearing support 400 of the frame 20 and an outer tubular member 410 of the bearing assembly 100, which also allow the control device 66 of the instrument 60 to be received within the bearing support 400 and the outer tubular member 410. The bearing support 400 may be provided instead of, or in addition to, the generally cylindrical bearing support 30 shown in Fig. 1. For example, the frame 20 may include the bearing support 400 shown in Fig. 23 for supporting the proximal end 104 of the bearing assembly 100 while the bearing support 30 shown in Fig. 1 may be provided to support the distal end 102 of the bearing assembly 100. Bearing assembly 100 may also be provided with one or more openings, slots, or similar apertures. Further, these openings may have optional coverings to prevent contamination or to help guide placement. These coverings could include a film, wrap, hinged door, sliding door, or similar device.

The bearing support 400 may be generally C-shaped or U-shaped such that the bearing support 400 forms a slot 402. The bearing assembly 100 may include a corresponding slot 412 that has similar dimensions (e.g., width and length) as the slot 402 in the bearing support 400. The slot 412 in the bearing assembly 100 may be formed in the outer tubular member 410 and in the bearing 130. The slots 402, 412 may be formed in an upper portion of the respective bearing support 400 and bearing assembly 100, as shown in Fig. 23. Accordingly, an instrument 60 having a control device 66 that extends upward may be inserted into the bearing assembly 100 such that the upward-extending control device 66 passes through the slots 402, 412. Also, the bearing assemblies 100 and bearing supports 400 allow easy loading of the respective instruments 60 even when the control device 66 of the respective instruments 60 is not passed through the slots 402, 412.

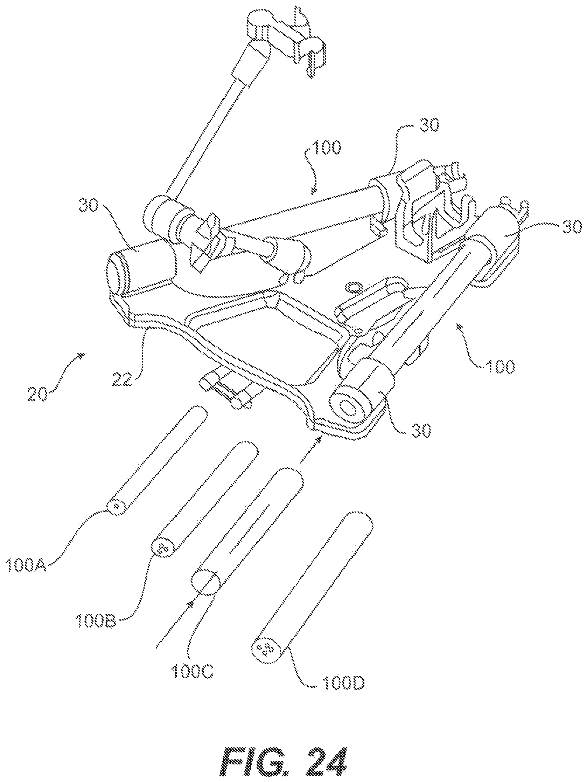

The frame 20 may accommodate various different configurations and sizes of bearing assemblies 100. For example, Fig. 24 shows an embodiment in which bearing assemblies 100a-100d of varying outer dimensions (e.g., different outer diameters, lengths, etc.) may be provided for a single frame 20. For example, as described above in connection with the embodiment shown in Fig. 21, the bearing support 200 may be adjustable to accommodate bearing assemblies 100a-100d of varying outer dimensions. Various cross-sections could be cylindrical, elliptical, triangular, curved, square, hexagonal, octagonal, or rectangular. Alternatively, or in addition, the bearing assemblies 100a-100d may also vary based on the dimensions (e.g., diameters) of the channels 116, 136, 156, other dimensions (e.g., length, wall thickness, etc.) or characteristics (e.g., materials, etc.) of the outer tubular member 110, the bearing 130, and/or the guide member 150, etc. Interchangeable outer tubular members 110 having varying outer diameters, inner diameters, and/or lengths may be provided. Also, the inner diameter of the bearing 130 may be variable. For example, the inner surface of the bearing 130 may be spring-loaded, inflatable, and/or electronically driven to adjust the inner diameter of the bearing 130, e.g., in the constriction 144. The inner diameter of the bearing 130 may be adjusted to accommodate instruments 60 of varying sizes. For example, in an embodiment, the sensor 206 may sense the size of the instrument 60, and a controller (not shown) may be used to determine and automatically adjust the inner diameter or configuration of the bearing 130 and/or a size, shape, and/or spacing of the rollers 202 in the bearing support 200.

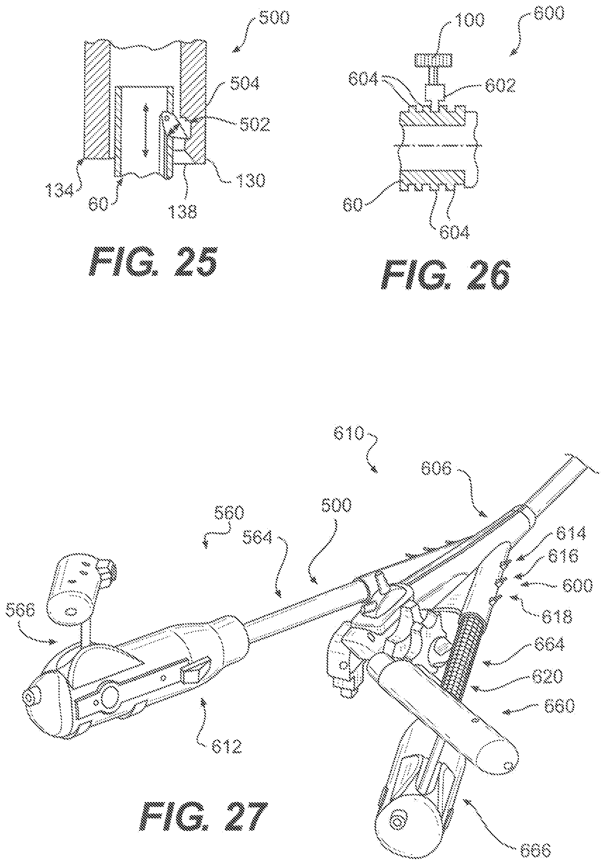

The bearing assembly 100 may include a locking mechanism to lock the instrument 60 in place rotationally and/or axially inside the bearing assembly 100. For example, Fig. 25 shows a locking mechanism 500 for positioning the instrument 60 with respect to the bearing assembly 100, according to an exemplary embodiment. An inner surface of one of the components of the bearing assembly 100, e.g., the outer tubular member 110, the bearing 130, or the guide member 150, may include a groove 502, and an outer surface of the instrument 60 that is configured to face the inner surface of the component of the bearing assembly 100 may include a pivotable latch 504. For example, as shown in Fig. 25, the groove 502 may be positioned in the inner surface of the bearing 130 near the proximal end 134. Locking mechanism 500 may include an axial lock or a rotational lock.

When the instrument 60 moves distally within the bearing assembly 100, the pivotable latch 504 is configured to slide against the inner surface of the bearing assembly 100 without catching the groove 502. Thus, the instrument 60 does not lock in place with respect to the bearing assembly 100. When the instrument 60 moves proximally within the bearing assembly 100, the pivotable latch 504 is configured to catch the groove 502 and prevent the pivotable latch 504 from moving proximal to the groove 502. Accordingly, the locking mechanism 500 may prevent the user from pulling the instrument 60 completely out of the bearing assembly 100.

Fig. 26 shows a locking mechanism 600 for positioning the instrument 60 with respect to the bearing assembly 100 according to another exemplary embodiment. The bearing assembly 100 (e.g., the outer tubular member 110, the bearing 130, or the guide member 150) may include an extendable member 602 that may be controlled to extend radially inward or retract radially outward within the channel 116, 136, 156 in the bearing assembly 100. For example, the extendable member 602 may be biased to extend radially inward until the user presses an actuator to release the extendable member 602 to retract the extendable member 602 radially outward. Alternatively, the extendable member 602 may be a screw that is rotatable in one direction to extend radially inward and rotatable in the opposite direction to retract radially outward. In some embodiments, a ratcheting peg may be used, which may be releasable. A screw, peg, cam, or similar device may create a friction fit to capture instrument 60. As another alternative, the extendable member 602 may be electronically driven. The outer surface of the instrument 60 may include a plurality of teeth 604 that are arranged in series along the length of the elongate member 64 of the instrument 60.

When the instrument 60 is in the bearing assembly 100, the user may extend the extendable member 602 radially inward to engage a slot between two adjacent teeth 604 on the instrument 60, thereby locking the instrument 60 in place within the bearing assembly 100. When the user wants to move the instrument 60 again, the user may retract the extendable member 602 from the slot between the teeth 604. Accordingly, the locking mechanism 600 allows the user to set the position of the instrument 60 within the bearing assembly 100 and to assist in preventing inadvertent movement of the instrument 60 with respect to the bearing assembly 100.

As a result, one or both of the locking mechanisms 500, 600 may be used to fix or lock the instrument 60 relative to the bearing assembly 100, which may free the user's hand to control other devices or instruments and which may reduce hand fatigue while keeping the instrument 60 ready for use. The locking mechanisms 500, 600 may also assist in preventing the removal of the instruments 60 from the sterile site and/or preventing the instruments 60 from falling out of the bearing assemblies 100.

Fig. 27 shows an endoscopy system 610 that may include one or more locking mechanism 500, 600, as previously described. In particular, one or more instruments 60 can include one or more locking mechanisms described herein. For example, an instrument 560 can include an actuator 612 that may be actuated to lock or unlock the locking mechanism 500. Locking mechanism 500 may be located on endoscopy system 610 at, for example, a support structure 606. Locking mechanism 500 can controllably limit movement of the elongate member 564 relative to the support structure 606. Actuator 612 can include a button, a thumb screw, a slide, a lever, or other type of manual or electronic switching device. One or more actuators could be located on endoscopy system 610 to control these or other locking mechanisms.

Endoscopy system 610 can also include one or more actuators 614, 616, 618 configured to control locking mechanism 600, as described above. For example, actuators 614, 616, 618 can be configured to selectively engage a grid surface 620 to control rotational movement, axial movement, and/or resistance to movement of an instrument 660. The grid surface 620 may be located on at least part of elongate member 664 or any other surface of endoscopy system 610.

Grid surface 620 can include a textured surface containing various grooves, ridges, and/or similar features. For example, grid surface 620 may include teeth similar to those shown and described in Fig. 26. Grooves or ridges of grid surface 620 can be straight, curved, or any suitable shape. Some features of grid surface 620 can be uniformly or randomly distributed.

In some instances, grid surface 620 may include a texture having a repeating pattern. For example, the repeating pattern could include a series of orthogonal grooves or ridges, helical grooves or ridges, or other uniform pattern. As described in detail below, a checked pattern of generally orthogonal grooves can provide selective axial and/or rotationally locking.

The repeating pattern may also include a series of features that vary in distribution. For example, a distribution of radial ridges or grooves may be larger in a distal section compared with smaller ridges or grooves in a proximal section. Such a distribution of ridges or grooves with different heights may serve to increase frictional forces opposing motion of the instrument 666 as the instrument 666 is moved distally. These and other configurations of grid surface 620 may be used to customize the operation of endoscopy system 610 for different users or applications.

In some embodiments, one or more pins (not shown), similar to extendable member 602 shown in Fig. 26, can engage grid surface 620. For example, actuator 614 may be actuated to cause one or more pins to engage a groove or ridge extending axially along the grid surface 620 and parallel to a longitudinal axis of the elongate member 664. Activating actuator 614 may permit axial movement of the instrument 666 relative to the support structure 606 while substantially locking rotational movement of the instrument 666 relative to the support structure 606. Deactivating actuator 614 may permit normal axial and rotational movement of the instrument 66 as described above.

In another example, the actuator 616 may cause another extendable member (now shown) to engage a groove or ridge extending radially or laterally about the elongate member 664. Activating actuator 616 may allow rotational movement of the instrument 666 relative to support structure 606 while substantially locking axial movement of instrument 666 relative to support structure 606.

In yet another example, the actuator 618 may be actuated to control a flexible elongate member (not shown) that may frictionally engage grid surface 620 and/or elongate member 664 to at least partially provide resistance to movement between the instrument 666 and the support structure 606. As described above, grid surface 620 could include features of varying size, depth, or height. These features could increase or decrease longitudinally and/or radially, providing increasing or decreasing resistance to movement.

Various locking or frictional systems can improve the operation of endoscopy system 610. Completely locking movement of one or more instruments during an operation can relieve a user's hand to reduce fatigue. Selectively locking axial, rotational, or other movement of an instrument can also reduce user fatigue and improve the precision of instrument movement. Various frictional settings can provide an indication of the instrument's positioning relative to the system or patient.

Relative movement between a bearing tube and the endoscopy system can control relative movement between the instrument and the patient or support structure. For example, the support structure could include a gear aligned axially or rotationally relative to the bearing tube. The gear could then be actuated to move the bearing tube axially or rotationally relative to the patient or support structure. Such relative movement could be combined with indexing to provided more precise instrument movement or positioning.

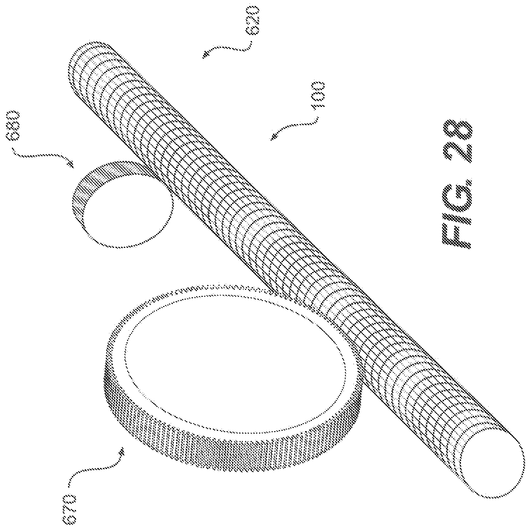

For example, Fig. 28 shows the bearing assembly 100 moveably coupled to a gear 670 configured for axial movement and a gear 680 configured for rotational movement. At least part of the bearing assembly 100 could include grid surface 620 as described above, wherein grid surface 620 can be configured to engage one or more gears 670, 680. In other embodiments, bearing assembly 100 could include one or more than two gears.

Gear 670 could engage and/or disengage grid surface 620 or bearing assembly 100. When engaged with grid surface 620, gear 670 could rotate to axially move bearing assembly 100. Gear 670 could also include a disk or a drum configured to frictionally engage bearing assembly 100 or other component of endoscopy system 610. Friction may be controlled based in part on a selection of materials that come into contact with each other, and may include, for example, a coating or covering on either component. At least partially locking the relative movement of one or more components can be controlled by friction, pressure, or other forces between interacting components.

Optionally, gear 680 can be positioned transverse or lateral to a longitudinal axis of bearing assembly 100. As described above for gear 670, gear 680 could engage grid surface 620 or frictionally engage bearing assembly 100. Gear 680 could rotate to rotatably move the bearing assembly 100 about its longitudinal axis. Such axial and/or rotational movement could provide precise linear control of bearing tube 100 or an instrument (not shown) contained within bearing tube 100.

In some embodiments, movement may be indexed to allow movement at specific distances. For example, axial movement may be indexed in centimeters or inches, and radial movement may be indexed in degrees. A stop, a preset distance, a visual marker, or an audible clicking mechanism may also be used to aid movement or alignment of a bearing assembly or instrument.

Gears 670, 680 may also include a spring (not shown) or other device configured to at least partially limit bearing assembly movement. A gear/spring assembly could provide at least partial locking of bearing assembly 100. Such movement restriction could provide another hands-free mechanism.

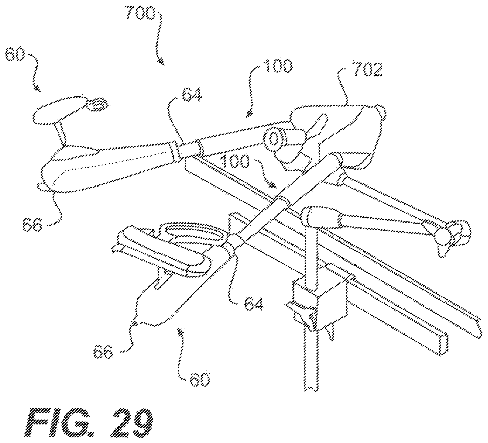

The bearing assembly 100 may be supported by the endoscopy system 10 in different ways. For example, instead of including bearing supports 30, 200, 300, 400, Fig. 29 shows an alternate embodiment of an endoscopy system 700 including the bearing assemblies 100 and a support structure 702 for locking both the bearing assemblies 100 in place and for supporting the bearing assemblies 100. For example, the support structure 702 may lock the guide member 150 of the bearing assembly 100 in place, e.g., rotationally and/or axially.

Bearing assembly 100 could further be modified as described below. For example, bearing assembly 100 could be formed from one or more coils configured to flex. Bearing assembly 100 may also be lined with low friction material, foam or otherwise be configured to reduce friction associated with the movement of instrument 60. Friction could also be reduced by using a ribbed, raised, or similarly lined inner surface of bearing assembly 100.

Bearing assembly 100 could also include one or more indices. For example, bearing assembly 100 could include a longitudinal index to provide a visual indication of the relative position between bearing assembly 100 and instrument 60. An angular index may provide a rotational reference of instrument 60 relative to bearing assembly 100 or frame 20.

The various components of the endoscopy system 10 described herein may be made of a suitable biocompatible material and may be flexible, for example, to traverse tortuous anatomy in the body. Any aspect set forth in any embodiment may be used with any other embodiment set forth herein. Every device and apparatus set forth herein may be used in any suitable medical procedure, may be advanced through any suitable body lumen and body cavity, and may be used to visualize, acquire, treat, or remove tissue from any suitable body portion.

It will be apparent to those skilled in the art that various modifications and variations can be made in the disclosed systems and processes without departing from the scope of the invention. Other embodiments of the invention will be apparent to those skilled in the art from consideration of the specification and practice of the invention disclosed herein. It is intended that the specification and examples be considered as exemplary only, with a true scope of the invention being indicated by the following claims and their equivalents

## Claims

1. A system for guiding an instrument, the system comprising:
a frame (20); and
a bearing assembly (100) supported by the frame and including:
an outer tubular member (110) supported by the frame and including a proximal end (104) and a distal end (102),
a bearing (130) at least partially disposed within the proximal end of the outer tubular member,
a guide member (150) at least partially inserted into the distal end (102) of the outer tubular member (110), and
a channel (116) for receiving the instrument, the channel extending through the outer tubular member, the bearing (130) and the guide member (150), wherein the channel (116) is defined by surfaces of each of the outer tubular member, the bearing and the guide member.

2. The system of claim 1, wherein the bearing (130) includes a distal end, a proximal end, and a constriction (144) in at least one of the distal end and the proximal end, and wherein the bearing includes a low friction inner surface defining at least a portion of the channel.

3. The system of claim 1, wherein the outer tubular member (110) is at least partially substantially transparent such that an interior of the channel is visible through a wall of the outer tubular member.

4. The system of claim 3, further comprising an elongate member (40) supported by the frame (20) and including a channel for receiving the instrument, wherein a distal end of the guide member (150) is configured to receive a proximal end of the elongate member.

5. The system of claim 1, wherein the frame (20) includes a bearing support (30, 200, 300, 400) for securing the bearing assembly to the frame.

6. The system of claim 5, wherein the bearing support (200) includes at least one roller (202) configured to move at least one of the instrument or the bearing assembly relative to the bearing support.

7. The system of claim 5, wherein one of the bearing assembly and the bearing support includes a first engaging member, and the other one of the bearing assembly and the bearing support includes a second engaging member for engaging with the first engaging member to position the bearing assembly relative to the bearing support.

8. The system of claim 1, wherein the bearing assembly includes a locking mechanism (500, 600) configured to lock the instrument (60) in place inside the bearing assembly.

9. The system of claim 1, wherein the bearing assembly includes an axial slot extending through the outer tubular member and the bearing, and extending distally from a proximal end of the bearing assembly.

10. The system of claim 8, further comprising a seal (52) formed at a connection between the distal end of the guide member and the proximal end of the elongate member (40) to assist in preventing fluids from exiting from within the bearing assembly and the elongate member at the connection between the bearing assembly and the elongate member.

11. The system of claim 1, wherein the elongate member (664) includes a grid surface (620) having at least one of a groove and a ridge, and the frame includes at least one actuator (618) configured to moveably engage the grid surface to limit movement of the elongate member relative to the bearing assembly.

12. The system of claim 11, wherein the grid surface includes orthogonal grooves and ridges, and the frame includes a first actuator configured to engage the grid surface to limit axial movement of the elongate member relative to the bearing assembly and a second actuator configured to engage the grid surface to limit rotational movement of the elongate member relative to the bearing assembly.

13. The system of claim 5, further comprising a drive mechanism connected to the bearing support (200) and configured to drive at least one of the instrument or the bearing assembly axially, wherein the drive mechanism includes a motor (204) configured to drive the at least one of the instrument or the bearing assembly axially.

14. The system of claim 13, wherein the drive mechanism includes at least one roller (202) configured to support the at least one of the instrument or the bearing assembly (100), the motor (204) being configured to rotate the at least one roller to drive the at least one of the instrument or the bearing assembly axially.

15. The system of claim 13, wherein the drive mechanism includes a sensor (206) configured to sense a position of the at least one of the instrument or the bearing assembly (100), and a controller configured to control the motor based on the sensed position.

## Patentansprüche

1. System zum Führen eines Instruments, wobei das System aufweist:
ein Gestell (20); und
eine Lageranordnung (100), die durch das Gestell gehalten wird und aufweist:
ein äußeres Röhrenelement (110), das durch das Gestell gehalten wird und ein proximales Ende (104) und ein distales Ende (102) aufweist,
ein Lager (130), das mindestens teilweise im proximalen Ende des äußeren Röhrenelements angeordnet ist,
ein Führungselement (150), das mindestens teilweise in das distale Ende (102) des äußeren Röhrenelements (110) eingesetzt ist, und
einen Kanal (116) zum Aufnehmen des Instruments, wobei sich der Kanal durch das äußere Röhrenelement, das Lager (130) und das Führungselement (150) erstreckt, wobei der Kanal (116) durch Oberflächen jeweils des äußeren Röhrenelements, des Lagers und des Führungselements definiert wird.

2. System nach Anspruch 1, wobei das Lager (130) ein distales Ende, ein proximales Ende und eine Verengung (144) im distalen Ende und/oder im proximalen Ende umfasst, und wobei das Lager eine reibungsarme Innenfläche aufweist, die mindestens einen Abschnitt des Kanals definiert.

3. System nach Anspruch 1, wobei das äußere Röhrenelement (110) mindestens teilweise im Wesentlichen transparent ist, so dass ein Inneres des Kanals durch eine Wand des äußeren Röhrenelements sichtbar ist.

4. System nach Anspruch 3, das ferner ein längliches Element (40) aufweist, das durch das Gestell (20) gehalten wird und einen Kanal zum Aufnehmen des Instruments umfasst, wobei ein distales Ende des Führungselements (150) konfiguriert ist, ein proximales Ende des länglichen Elements aufzunehmen.

5. System nach Anspruch 1, wobei das Gestell (20) eine Lagerhalterung (30, 200, 300, 400) zum Befestigen der Lageranordnung am Gestell aufweist.

6. System nach Anspruch 5, wobei die Lagerhalterung (200) mindestens eine Rolle (202) aufweist, die konfiguriert ist, das Instrument und/oder die Lageranordnung relativ zur Lagerhalterung zu bewegen.

7. System nach Anspruch 5, wobei eine der Lageranordnung und der Lagerhalterung ein erstes Eingriffselement aufweist, und die andere der Lageranordnung und der Lagerhalterung ein zweites Eingriffselement aufweist, um mit dem ersten Eingriffselement in Eingriff zu treten, um die Lageranordnung relativ zur Lagerhalterung zu positionieren.

8. System nach Anspruch 1, wobei die Lageranordnung einen Verriegelungsmechanismus (500, 600) aufweist, der konfiguriert ist, das Instrument (60) an seinem Ort innerhalb der Lageranordnung zu verriegeln.

9. System nach Anspruch 1, wobei die Lageranordnung einen axialen Schlitz aufweist, der sich durch das äußere Röhrenelement und das Lager erstreckt und sich distal von einem proximalen Ende der Lageranordnung erstreckt.

10. System nach Anspruch 8, das ferner eine Dichtung (52) aufweist, die an einer Verbindung zwischen dem distalen Ende des Führungselements und dem proximalen Ende des länglichen Elements (40) ausgebildet ist, um dabei zu helfen, das Austreten von Fluiden aus der Lageranordnung und dem länglichen Element an der Verbindung zwischen der Lageranordnung und dem länglichen Element zu verhindern.

11. System nach Anspruch 1, wobei das längliche Element (664) eine Gitteroberfläche (620) aufweist, die eine Rille und/oder einen Grat aufweist, und das Gestell mindestens ein Betätigungselement (618) aufweist, das konfiguriert ist, beweglich mit der Gitteroberfläche in Eingriff zu treten, um eine Bewegung des länglichen Elements relativ zur Lageranordnung zu begrenzen.

12. System nach Anspruch 11, wobei die Gitteroberfläche orthogonale Rillen und Grate aufweist, und das Gestell ein erstes Betätigungselement, das konfiguriert ist, mit der Gitteroberfläche in Eingriff zu treten, um eine axiale Bewegung des länglichen Elements relativ zur Lageranordnung zu begrenzen, und ein zweites Betätigungselement aufweist, das konfiguriert ist, mit der Gitteroberfläche in Eingriff zu treten, um eine Rotationsbewegung des länglichen Elements relativ zur Lageranordnung zu begrenzen.

13. System nach Anspruch 5, das ferner einen Antriebsmechanismus aufweist, der mit der Lagerhalterung (200) verbunden und konfiguriert ist, das Instrument und/oder die Lageranordnung axial anzutreiben, wobei der Antriebsmechanismus einen Motor (204) aufweist, der konfiguriert ist, das Instrument und/oder die Lageranordnung axial anzutreiben.

14. System nach Anspruch 13, wobei der Antriebsmechanismus mindestens eine Rolle (202) aufweist, die konfiguriert ist, das Instrument und/oder die Lageranordnung (100) zu halten, wobei der Motor (204) konfiguriert ist, die mindestens eine Rolle zu drehen, um das Instrument und/oder die Lageranordnung axial anzutreiben.

15. System nach Anspruch 13, wobei der Antriebsmechanismus einen Sensor (206), der konfiguriert ist, eine Position des Instruments und/oder der Lageranordnung (100) abzutasten, und eine Steuereinrichtung aufweist, die konfiguriert ist, den Motor beruhend auf der abgetasteten Position zu steuern.

## Revendications

1. Système de guidage d'un instrument, ledit système comprenant :
un cadre (20) ; et
un dispositif de support (100) monté sur le cadre et comportant :
un élément tubulaire extérieur (110) supporté par le cadre et ayant une extrémité proximale (104) et une extrémité distale (102),
un palier (130) disposé au moins en partie dans l'extrémité proximale de l'élément tubulaire extérieur,
un élément de guidage (150) inséré au moins en partie dans l'extrémité distale (102) de l'élément tubulaire extérieur (110), et
un canal (116) destiné à recevoir l'instrument, ledit canal s'étendant dans l'élément tubulaire extérieur, le palier (130) et l'élément de guidage (150), ledit canal (116) étant défini par des surfaces de l'élément tubulaire extérieur, du palier et de l'élément de guidage.

2. Système selon la revendication 1, où le palier (130) présente une extrémité distale, une extrémité proximale et un rétrécissement (144) dans l'extrémité distale et/ou dans l'extrémité proximale, et où le palier comporte une surface intérieure à faible friction définissant au moins une partie du canal.

3. Système selon la revendication 1, où l'élément tubulaire extérieur (110) est au moins en partie sensiblement transparent, de manière à rendre visible l'intérieur du canal au travers d'une paroi de l'élément tubulaire extérieur.

4. Système selon la revendication 3, comprenant en outre un élément allongé (40) supporté par le cadre (20) et comprenant un canal destiné à recevoir l'instrument, une extrémité distale de l'élément de guidage (150) étant prévue pour recevoir une extrémité proximale de l'élément allongé.

5. Système selon la revendication 1, où le cadre (20) comprend un support de palier (30, 200, 300, 400) destiné à fixer le dispositif de support au cadre.

6. Système selon la revendication 5, où le support de palier (200) comprend au moins un rouleau (202) prévu pour déplacer l'instrument et/ou le dispositif de support par rapport au support de palier.

7. Système selon la revendication 5, où une unité entre le dispositif de support et le support de palier comprend un premier élément d'engagement, et où l'autre unité entre le dispositif de support et le support de palier comprend un deuxième élément d'engagement destiné à s'accoupler avec le premier élément d'engagement pour positionner le dispositif de support par rapport au support de palier.

8. Système selon la revendication 1, où le dispositif de support comprend un mécanisme de verrouillage (500, 600) prévu pour fixer l'instrument (60) en place à l'intérieur du dispositif de support.

9. Système selon la revendication 1, où le dispositif de support présente une fente axiale s'étendant dans l'élément tubulaire extérieur et le palier, et s'étendant distalement depuis une extrémité proximale du dispositif de support.

10. Système selon la revendication 8, comprenant en outre un joint (52) formé sur un raccord entre l'extrémité distale de l'élément de guidage et l'extrémité proximale de l'élément allongé (40) pour empêcher l'écoulement de fluides hors du dispositif de support et de l'élément allongé à la connexion entre le dispositif de support et l'élément allongé.

11. Système selon la revendication 1, où l'élément allongé (664) présente une surface structurée (620) pourvue d'une rainure et/ou d'une nervure, et où le cadre comporte au moins un actionneur (618) prévu pour s'engrener de manière mobile avec la surface structurée afin de limiter le déplacement de l'élément allongé par rapport au dispositif de support.

12. Système selon la revendication 11, où la surface structurée présente des rainures et des nervures orthogonales, et où le cadre comporte un premier actionneur prévu pour s'engrener avec la surface structurée afin de limiter le déplacement axial de l'élément allongé par rapport au dispositif de support, et un deuxième actionneur prévu pour s'engrener avec la surface structurée afin de limiter la rotation de l'élément allongé par rapport au dispositif de support.

13. Système selon la revendication 5, comprenant en outre un mécanisme d'entraînement raccordé au support de palier (200) et prévu pour entraîner axialement l'instrument et/ou le dispositif de support, ledit mécanisme d'entraînement comprenant un moteur (204) prévu pour entraîner axialement l'instrument et/ou le dispositif de support.

14. Système selon la revendication 13, où le mécanisme d'entraînement comprend au moins un rouleau (202) prévu pour supporter l'instrument et/ou le dispositif de support (100), ledit moteur (204) étant prévu pour faire tourner ledit au moins un rouleau afin d'entraîner axialement l'instrument et/ou le dispositif de support.

15. Système selon la revendication 13, où le mécanisme d'entraînement comprend un capteur (206) prévu pour détecter la position de l'instrument et/ou du dispositif de support (100), et un dispositif de commande prévu pour commander le moteur en fonction de la position détectée.
